# EUROPEAN PATENT APPLICATION

(11) **EP 0 703 451 A2**
(43) Date of publication of application: **27.03.1996**
(21) Application number: 95305890.6
(22) Date of filing: 23.08.1995
(51) Int. Cl.: G01N 33/34, G01N 21/64

(54) **Monitoring process fluids in papermaking systems**

(30) Priority: 26.09.1994 US 312021
(71) Applicant: NALCO CHEMICAL COMPANY, Naperville Illinois 60563-1198 (US)
(72) Inventor: Tubergen, Karen B., Mt Prospect, Illinois 60056 (US)
(74) Representative: Harrison, David Christopher

(57) **Abstract**

A method of monitoring a papermaking system containing impurities and containing a dosage of a treating component to determine the level of treating under operating conditions is disclosed which adds to the papermaking system a water soluble fluorescent tracer in an amount proportioned to the amount of treating component in the system, the fluorescent tracer being inert to the papermaking environment. A sample solution is withdrawn from the system containing both the component and tracer and the withdrawn sample is subjected to an analysis which comprises the step of comparing the tracer concentration to a standard to determine the concentration of tracer in the sample. After determining the variance of the treating component dosage as compared to the standard, the dosage is adjusted by changing the volume of water or the dosage of treating component in the papermaking system to maintain an acceptable operating range of concentration for the treating component. The method may be used for detecting leakage from the system.

## Description

The present invention relates to the monitoring of process fluids in papermaking systems, optionally together with an appropriate responsive treatment of the and, more particularly, to the utilization of compositions containing fluorescent agent(s) as a means of detecting leaks in such systems, as well as to quantify and control fee rate(s) of treatment chemicals into papermaking systems.

In a papermaking system, the aqueous system is comprised of at least one body of process fluid, serviced by at least one body of temperature-conditioning fluid, which is commonly an aqueous based temperature-conditioning water, such as cooling water. These bodies are held within separate lines or other confines to prevent contamination of one body with the fluid of the other. The servicing of the process fluid by the temperature-conditioning fluid however generally requires such fluids to be brought into close proximity, such as being routed on opposite sides of a heat exchanger wall or surface. The close proximity of such distinct bodies of fluids creates a serious, but unavoidable, risk of leakage between bodies, whereby at least one body is contaminated by the fluid of the other. The ramifications of such process fluid/temperature-conditioning fluid leakage can be extremely serious because the makeups of such fluids are very dissimilar in most instances.

Temperature-conditioning fluids routinely are waters that contain far less solutes or other non-H₂0 substances than process fluids. The non-H₂0 substances of temperature-conditioning waters are typically inorganic materials, and organic substances, whether naturally occurring or added as treatment chemicals, are present only in relatively minute amounts. In contrast, not only does the concentration of non-H₂0 substances in process fluids typically dwarf that of temperature-conditioning fluids, such non- H₂0 substances often are comprised in significant portion of organic materials.

Leakage from process fluid to temperature-conditioning fluid not only represents a process fluid loss, which can significantly increase the overall manufacturing costs and can lead to serious fouling of the temperature-conditioning fluid system. Leakage from temperature-conditioning fluid to process fluid not only represents a temperature-conditioning fluid loss, which can throw off a temperature-conditioning fluid treatment program, but also can seriously taint the process fluids. These dissimilar fluids are not intended to contact each other or intermix in any manner, and when the mingling of some portion of one with the other occurs through leakage, a method whereby such leakage is not only detected, but also located and/or quantified, would be highly advantageous to the manufacturing process.

It is an object of the present invention to provide a method for detecting leakage between at least one body of temperature-conditioning fluid and at least one body of process fluid in a papermaking process. It is an object of the present invention to provide a method for locating the site of Leakage between at least one body of temperature-conditioning fluid and at least one body of process fluid in an industrial process. It is an object of the present invention to provide a method for quantifying Leakage between at least one body of temperature-conditioning fluid and at least one body of process fluid in an industrial process. It is an object of the present invention to provide a method for responsive treatment of a body of temperature-conditioning fluid and/or a body of process fluid upon the detection, location and/or quantification of leakage there between in an industrial process. These and other objects of the present invention are discussed in detail below.

In a papermaking system to which a treating component is added, maintaining the proper feed level for the component is essential for optimal performance. Improper feed rate of treating component can lead to serious problems. One common method of estimating the concentration of a treating component focuses on measuring the level of an active component in the treatment formulation (e.g. polymeric drainage aids and retention aids). That technique is often unsatisfactory due to one or more of the following problems: background interference's from the system liquid or materials contained in the liquid; analytical methods use bulky and costly equipment; time-consuming, labor-intensive analyses are not compatible with continuous monitoring and inaccurate readings result from degradation or deposition of active components within the system.

The invention relates to on-stream monitoring of the level of a treating component added to a papermaking system. The treating component is added to improve the quality of the water such as by the use of retention aids and drainage aids.

A non-consumable or system-inert tracer can be proportioned to the treating component in terms of initial concentration added to the system. This is the standard. If the treating component is added at a rate equal to or greater than the recommended rate, as it should be, the concentration of tracer to treating component will increase proportionally. This increase can be compared to the recommended or theoretical standard to determine performance, i.e., whether the sample to standard color intensity comparison shows the treating component is present at the expected concentration. If not, there are several possibilities: there is an undertreatment to the detriment of the equipment, there is an overtreatment which is a waste, or water in the system is being unexpectedly lost along with the treatment chemical. The first two possibilities involve a correction in the dosage of treating component. The third possibility calls for a system audit.

The feed rate (dosage) of chemical treatment is commonly an estimate which in turn depends upon several complex and variable factors. Under changing operating conditions, the dosage will also change, and hence the need for accurate, precise monitoring of the tracer. The system may respond at different rates to the dosage change, until equilibrium is reached.

U.S. Patent No. 4,783,314, issued to John Hoots, presents a thorough analysis of the use of a fluorescent tracer to monitor treating component performance in cooling water systems. Pending application Ser. No. 258,131, filed October 14, 1988, discloses instrumentation by which monitoring can be conducted continuously. The disclosures of these references are hereinafter incorporated by reference.

The tracer must be transportable in the waste system without change. In actual use, the treating component will be consumed. If actual performance of the treating agent conforms to the theoretical performance (postulated rate of consumption) the ratio of tracer to treating component will increase. Thus, the tracer, to serve as an index of the product or treating component performance must fulfill several criteria. Firstly, selected chemicals must be detectable on a continuous or semicontinuous basis. Measurements of concentration must be accurate, repeatable and capable of being performed on many different waters (i.e. clean, turbid, hard, soft, etc.). Secondly, the tracer material cannot be one already present in a significant quantity in the water used for industrial cooling. Thirdly, testing for the tracer cannot be interfered with, or biased, by other chemical compounds normally present in cooling water. Fourth, the tracer must not reduce the efficacy of such active ingredients of the treatment chemicals themselves. Fifth, since the tracer must be added with the treating component, the material selected as the tracer must be compatible with the actives (treating components) with respect to formulation, storage, freeze-thaw recovery, etc. Finally, the tracer cannot be toxic, or represent any sort of environmental problem upon discharge. Ideally, any material used in the tracer role would be completely biodegradable.

### Summary of the Invention

The present invention claims a method of monitoring a papermaking system containing impurities and containing a dosage of a treating component to determine the level of treating under operating conditions comprising the adding to the papermaking system a water soluble fluorescent tracer in an amount proportioned to the amount of treating component in the system, the fluorescent tracer being inert to the papermaking environment. A sample solution is withdrawn from the system containing both the component and tracer and the withdrawn sample is subjected to an analysis which comprises the step of comparing the tracer concentration to a standard to determine the concentration of tracer in the sample. After determining the variance of the treating component dosage as compared to the standard, the dosage is adjusted by changing the volume of water or the dosage of treating component in the papermaking system to maintain an acceptable operating range of concentration for the treating component.

A further embodiment of the invention comprises providing instrumentation including an analyzer having a flow cell for receiving a sample of circulating water from the papermaking system and for sensing a characteristic of the tracer indicative of its concentration, the analyzer having a transducer for converting the characteristic to a voltage analog thereof; a monitor for receiving said voltage analog and for continuously comparing it to a voltage standard representing a standard tracer in consumption of the treating component, the monitor generating an output signal when the comparison establishes nonstandard performance; and a pump unit for introducing the proportioned treating component and tracer at a predetermined dosage, the output signal controlling the pump unit to alter the dosage of treating component.

The present invention further provides a method for detecting leakage between an industrial process fluid and a temperature-conditioning fluid in a papermaking comprising at least a sample of at least one of such fluids to analysis to detect the presence and/or the concentration of a signature compound therein, and al times the concentration change and/or concentration gradient of the signature compound in at least one of such fluids. In a preferred embodiment at least one of such fluids is an aqueous-based fluid. In another preferred embodiment the analysis is a fluorescence analysis or a combination of high pressure liquid chromatography and fluorescence analysis.

### Description of the Preferred Embodiments

In general, the concentration of a fluorescent tracer is directly determined from a calibration curve of tracer concentration versus emission. That comparison permits the determination of the concentration range over which linear emission response is observed. At higher tracer concentrations, a negative deviation from ideal behavior is observed. The concentration of the tracer can still be determined directly from the calibration curve or the sample can simply be diluted until the tracer concentration falls within the linear emission response range. For extremely dilute samples, techniques exist for increasing the concentration of the fluorescent tracer (e.g. liquid-liquid extraction) until it lies within a desirable concentration range.

By properly choosing the fluorescing recomponent, quantitative and *in situ* measurement of tracer levels from parts per trillion (ppt) to parts per million (ppm) can be routinely accomplished on an instant or continuous basis with inexpensive portable equipment. In addition, multiple tracers may be used concurrently by choosing tracers with proper spectral characteristics. As such, various combinations of fluorescent tracers and treatment feeds can be quantified within a papermaking system. For example, four individual treatments containing a single unique fluorescent tracer plus one additional treatment containing the two fluorescent tracers could be employed within a papermaking system. In that case, each fluorescent tracer and the corresponding individual concentration of the five treatments can each be quantified. In addition to being able to quantify complex combinations of the treatment feeds, fluorescent compounds are available which are environmentally acceptable and are not degraded by or deposited within the papermaking systems, and are available at low cost.

The invention can generally be applied in the following ways:
(a) direct addition of from one or more fluorescent tracers to a papermaking system:
(b) incorporation of one to six (or even more) fluorescent tracers into a chemical treatment composition containing other components and said treatment is applied to papermaking system in order to maintain proper operation of that system;
(c) addition of one to six chemical treatment components (or even more) containing fluorescent tracer(s) directly into papermaking system or into liquid feed leading into system;
(d) addition of fluorescent tracers so that within the papermaking system individual tracer concentrations ranging from 1 part per trillion to 100 parts per million (ppm), preferably from 1 part per billion (ppb) to 10 ppm, and most preferably from 10 ppb to 2 ppm are realized.

A low concentration of tracer is desirable in order to assure that the tracer is widely dispersed and effectively isolated from disturbances, as compared to high concentrations where the chances of losing linear detector response versus trace level are increased.

One object of the present invention is to employ a tracer, selected from the class of transition metals, to determine treating component performance, and, at the same time, permit the operator to determine quantitatively other uncompensated chemical stresses.

Consequently, it becomes possible to monitor the treating component level for par performance (adjusting the dosage if needed) and, separately, to then identity any prevailing chemical or operating condition stress while adjusting the product dosage accordingly.

The tracer will have been added to the system water in a proportioned amount with the treating component. The amount of tracer in the treating component is typically 0.5% by weight, or 0.5 ppm at a treating component level of 100 ppm.

The system sample to be monitored is passed through an on-line analyzer, which performs an analysis automatically, displays the tracer concentration in ppm, produces a corresponding control voltage to the pump which supplies the treating component if its concentration is out of the set range and imposes a further control on the pump if stressing is detected.

Colorimetry or spectrophotometry may be employed using a Brinkman Pc-8ol probe colorimeter (570 nm filter). The sample solution is admitted to a flow cell in which a fiber optic (dual) probe is immersed. One fiber optic cable shines incident light through the process liquid onto a mirror inside the cell and reflected light is transmitted back through the process liquid into a fiber optic cable and then to the colorimetric analyzer unit by the other cable as shown by arrows. The colorimeter has a transducer which develops an electrical analog signal of the reflected light characteristic of the tracer concentration. The voltage emitted by the transducer activates a dial indicator and a continuous line recorder printout unit. A set point voltage monitor (not shown, but as in the foregoing embodiments) will constantly sense (monitor) the voltage analog generated by the colorimeter and if nonstandard performance is established a signal is transmitted to alter accordingly the feed rate of the pump supplying the chemical treatment.

An ion selective electrode may be employed to determine the concentration of an inert tracer ion (K+ is a good example) in terms of the relationship between the electrical signal developed by the electrode and the concentration of tracer. By calibration (potential or current vs. concentration) the ionic concentration at the sample electrode can be indexed to a reference (standard) electrode which is insensitive to the tracer ion. To provide continuous monitoring of the tracer, the electrodes may be dipped directly into a flowing stream of the cooling water, collectively constituting a flow cell, or the cooling water could be passed through an external flow cell into which the ion-selective and reference electrodes have been inserted.

The present method of continuous monitoring and resultant feedback to control the feed rate of the treating component allows for additional diagnostic supervision in addition to the feedback control just mentioned. By following the recorder or hard print-out records, aberrations, anomalies and system faults are readily perceived and can be corrected as the case may be. Examination of the performance record on the first day (after allowing for system equilibration) may reveal consumption of ten pounds of treating component, for example, whereas the second day record may show twenty pounds (to continue the example) even though the product concentration was being held constant based on monitoring the tracer. A divergence in product usage of this scale would demand investigation, and the investigation may reveal development of a serious leak in the system, not a mere aberration or anomaly, requiring a correction to cure the water loss.

The continuous monitor record may reveal high or low concentration anomalies of short duration which can be ignored. On the other hand, high or low treatment corrections of appreciable duration or continuity cannot be ignored, requiring investigation. If the record establishes need for more, or less, treating component than was thought, the rate of change can be estimated or even calculated by a differential equation since the print-out establishes both time and concentration changes. Thus, the operator can estimate or calculate the increase or decrease in product feed rate, the more readily to restore the system to balance, and thereafter for the steady state rate of feed.

Use of a single inert tracer may be employed to monitor a brace of treating when the two treating components are fed proportionally, say a high rate pump feeding of one product at twice the rate of a second product dosed by a pump whose feed rate is directly tied (slaved) to the first. Since the tracer will be proportioned to one of the two treating components, it is, by definition under this invention, proportioned to the other. The invention represents a considerable advance over the current practice of taking grab samples, running them to the analysis room, waiting for the analytical report and then manually adjusting a knob on a pump controller. Grab samples, at best, must be averaged to be interpreted and when interpreted represent past performance some time ago not present performance on an instantaneous real-time basis which in part characterizes the present invention.

Temperature-conditioning fluids include cooling waters, such as once-through cooling waters, recirculating cooling waters, other indirect contact cooling waters, heating waters, such as pasteurization waters, and the like. Temperature-conditioning fluids and process fluids are generally brought into indirect contact for the purpose of heat transfer from the source fluid to the receiver, during which heat transfer process the fluids are separated by a barrier that is a good conductor of heat, which barrier is called the heat transfer surface. An assembly comprised of at least one heat transfer barrier, which barrier has a process fluid side and a cooling fluid side, in a containment vessel, is called a heat-exchanger. A simple heat-exchanger may be comprised of a tube or pipe located concentrically inside of another (the shell). In such a shell-and-tube heat-exchanger the process fluid typically flows through the inner pipe, and the cooling fluid flows through the annulus between the inner pipe and the shell. In a parallel or concurrent flow heat-exchanger, both fluid streams flow in the same direction. In a countercurrent flow heal-exchanger, the fluid streams flow in opposite directions. Regardless of the type or complexity of a heat-exchanger, a characteristic common to all is transfer of heat across a heat transfer surface that has a process fluid side and an opposite cooling fluid side.

Leakage between a temperature-conditioning fluid and a process fluid most normally occurs within a heat-exchanger because within a heat-exchanger there is generally only a single heat exchange surface or wall separating the fluid bodies. Moreover, there generally is a temperature gradient across such surface or wall and a significant stream flow along at Ieast one side of such surface or wall, which conditions create material stresses that can lead to ruptures.

Regardless of how leakage occurs, the dissimilarity between such fluids generally is a controlling factor as to the gravity of the problem(s) created by cross-contamination. Since papermaking system waters are often treated with corrosion inhibitors, scale inhibitors, possibly biocides, and other agents, even extremely minute amounts of these agents may be sufficient to seriously taint the material in the process waters. Cross-contamination between temperature-conditioning fluids and process fluid can lead to such grave problems that the choice of temperature-conditioning fluid treatment agents may be limited to those agents having less potential for harm but less than optimum treatment effectiveness. The process of the present invention may in some instances open such limitations to more effective agents.

In addition in an embodiment of the present invention the process includes a responsive activation of an appropriate treatment of the contaminated fluid upon the detection and/or quantification of leakage thereinto. Thus in a preferred embodiment of the invention, the detection and/or quantification of a leakage that releases a contaminant to one of the fluids also activates a feed of at least one treatment agent, effective to decrease the deleterious effect of such contamination, to the contaminated fluid. In further preferred embodiment, such responsive activation of treatment agents also dictates that an amount of such treatment agent effective to decrease the deleterious effect of such contamination is fed to the contaminated fluid.

Cooling waters prior to process fluid contamination generally can be characterized as having a pH of from about 6.5 to about 9.5, having a concentration of solutes of up to about 10,000 ppm, and having a concentration of suspended, or colloidal, solids of not more than up to about 200 ppm. Process fluids in a papermaking system are even less susceptible to makeup characterizations than cooling waters. Process fluids are often, but not always, organic based, and generally have a wide concentration range of non-H₂O material, including solutes, colloidal and suspended solids, and non-miscible fluids. While many process fluids contain organics, and a major concern may be contamination of the temperature-conditioning fluid with such organics, in other industrial water process the process fluids may instead contain inorganic material which is a major concern as to temperature-conditioning fluid contamination.

The signature compound(s) is a tagged version of a normal component of one of the fluids, for instance a polymeric cooling water treatment agent tagged with fluorescent group(s). The tracer is preferably selected from among those that are easily detectable by the analysis method being employed in the process. Such analysis methods include fluorescence analysis, HPLC and fluorescence analysis combinations, ion-electrode analysis, colorimetry analysis, transition metal analysis, combinations of HPLC and other detection methods such as light absorbance analysis, post-column derivatization, conductivity and the like, some of which are described in more detail below.

### Fluorescence Emission Spectroscope

The detection and quantification of specific substances by fluorescence emission spectroscopy is founded upon the proportionality between the amount of emitted light and the amount of a fluoresced substance present. When energy in the form of light, including ultraviolet and visible light, is directed into a sample cell, fluorescent substances therein will absorb the energy and then emit that energy as light having a longer wavelength than the absorbed light.

The amount of emitted light is determined by a photodetector. In practice, the light is directed into the sample cell through an optical light filter so that the light transmitted is of a known wavelength, which is referred to as the excitation wavelength and generally reported in nanometers ("nm"). The emitted light is similarly screened through a filter so that the amount of emitted light is measured at a known wavelength or a spectrum of wavelengths, which is referred to as the emission wavelength and generally also reported in nanometers. When the measurement of specific substances or categories of substances at low concentrations is desired or required, such as often is the case for the process of the present invention, the filters are set for a specific combination of excitation and emission wavelengths, selected for substantially optimum low-level measurements.

Fluorescence emission spectroscopy is one of the preferred analysis techniques for the process of the present invention. Some water treatment agents may be susceptible to tagging with fluorescent groups, for instance as disclosed in U.S. Patent No. 5,128, 419, D. W. Fong and J. E. Hoots, issued July 7, 1992, the disclosure of which is incorporated herein by reference, wherein the tagging of polymers with pendant fluorescent groups by (trans)amidation derivatization of pre-existing polymers having carbonyl-type pendant groups is disclosed. Water treatment polymers tagged with pendant fluorescent groups may of course be prepared by methods other than (trans)amidation derivatization. Other fluorescent chemical tracers and monitoring techniques are now known, for instance as disclosed in U.S. Patent No. 4,783,314, I. E. Hoots and B. E. Hunt, issued November 8, 1988, the disclosure of which is incorporated herein by reference, wherein inert fluorescent tracers are employed in combination with a fluorescence monitoring, such as the sodium salt of 2-naphthalenesulfonic acid and Acid Yellow dye.

In general for most fluorescence emission spectroscopy methods having a reasonable degree of practicality, it is preferable to perform the analysis without isolating in any manner the fluorescent tracer. Thus there may be some degree of background fluorescence. In instances where the background fluorescence is low, the relative intensities (measured against a standard fluorescent compound at a standard concentration and assigned a relative intensity for instance l 00) Or the fluorescence Or the tracer versus the background can be very high, for instance a ratio of 100/10 or 500/10 while certain combinations of excitation and emission wavelengths are employed even at low fluorescent compound concentrations, and such ratios would be representative of relative performance (under like conditions) of respectively 10 and 50. For most cooling water backgrounds, a compound that has a relative performance of at least about 5 at a reasonable concentration is very suitable as a fluorescent tracer itself or as a tagging agent for water treatment polymers and the like when such compounds contain an appropriate reactive group for the tagging reaction. When there is or may be a specific chemical specie of reasonably high fluorescence in the background, the tracer and the excitation and/or emission wavelengths often can be selected to nullify or at least minimize any interference of the tracer measurement(s) caused by the presence of such specie.

One method for the continuous on-stream monitoring of chemical tracers by fluorescence emission spectroscopy and other analysis methods is described in U.S. Patent No. 4,992,380, B. E. Moriarity, J. J. Hickey, W. H. Hoy, J. E. Hoots and D. A. Johnson, issued February 12, 1991, the disclosure of which is incorporated herein by reference.

### Combined HPLC-Fluorescence Analysis

The combination of high-pressure liquid chromatography ("HPLC") and fluorescence analyses of fluorescent tracers is a powerful tool for the present leak detection process, particularly when very low levels of the fluorescent tracer are used or the background fluorescence encountered would otherwise interfere with the efficacy of the fluorescence analysis. The HPLC-fluorescence analysis method allows the tracer compound to be separated from the fluid matrix and then the tracer concentration can be measure. The combination of HPLC-Fluorescence analysis is particularly effective for measuring minute levels of tracer in highly contaminated fluids.

The HPLC method can also be effectively employed to separate a tracer compound from a fluid matrix for the purposes of then employing a tracer-detection method other the fluorescence analysis, and such other tracer-detection methods include without limitation light absorbance, post-column derivatization, conductivity and the like.

### Colorimeter Analysis

Colorimetry or spectrophotometry may be employed to detect and/or quantify a chemical tracer. Colorimetry is a determination of a chemical specie from its ability to absorb ultraviolet or visible light. One colorimetric analysis technique is a visual comparison of a blank or standard solution (containing a known concentration of the tracer specie) with that of a sample of the fluid being monitored. Another colorimetric method is the spectrophotometric method wherein the ratio of the intensities of the incident and the transmitted beams of light are measured at a specified wavelength by means of a detector such as a photocell or photomultiplier tube. Using a colorimetric probe, a fiber optic (dual) probe, such as a Brinkman PC-80 probe (570 nm filter), a sample solution is admitted to a flowcell in which the probe is immersed. One fiber optic cable shines incident light through the sample liquid onto a mirror inside the cell and reflected light is transmitted back through the sample liquid into a fiber optic cable and then to the colorimetric analyzer unit, which contains a colorimeter, by the other cable. The colorimeter has a transducer that develops an electrical analog signal of the reflected light characteristic of the tracer concentration. The voltage emitted by the transducer activates a dial indicator and a continuous line recorder printout unit. A set point voltage monitor may be employed to constantly sense or monitor the voltage analog generated by the colorimeter, and upon detection of a tracer signal (discussed below), a responsive signal may be transmitted to a responsive treatment agent feed line to commence or alter the rate of feed. Such a colorimetric analysis technique and the equipment that may be employed therefor are described in U.S. Patent No. 4,992,380, B. E. Moriarity, J. J. Hickey, W. H. Hoy, J. E. Hoots and D. A. Johnson, issued February 12, 1991, incorporated hereinto by reference. Chemical tracers suitable for use in conjunction with a colorimetric technique include transition metals (discussed below) and substances which show light absorbance which is detectable from that of other species present in the system fluid or substances which react with color-forming reagents to produce light absorbance which is detectable from that of other species present in the system fluid.

### Ion Selective Electrode Analysis

An ion selective electrode may be used to determine the concentration of an inert chemical tracer through the direct potentiometric measurement of specific ionic tracers in aqueous systems. These electrodes respond only to selected ionic materials and gases dissolved in liquids, and hence such tracers must be ionized (or dissolved gases) in the environment in which they are to be determined. Ion selective electrodes work like pH electrodes, depending on a potential developed across a thin membrane by the difference in the concentrations of the ion (or gas) to be measured on each side of the ionically conducting thin layer. The concentration within the electrode is fixed and the potential varies with(h the concentration of ions (or gas) in the same. By calibration (the potential or current versus the concentration), the ionic (or gas) concentration at the sample electrode can be indexed to a reference or standard electrode that is insensitive to the tracer ion. To provide continuous monitoring of the tracer, the electrodes may be dipped directly into a stream of one of the fluids (collectively comprising a flow cell), or the fluid being monitored may be passed through an external Row cell into which the ion-selective and reference electrodes have been inserted. An ion selective electrode tracer monitoring technique and the equipment therefor are described in US-A-4,992,380.

### Transition Metal Analysis

A transition metal compound (transition metal ions, oxy-anions, cations and associated complexes) can be quantitatively measured by one or more of known techniques. A preferred technique is the colorimetry analysis discussed above. Another technique is molecular absorption. Molecular absorption in the ultra violet and visible region depends on the electronic structure of the molecule. The energy absorbed elevates electrons from orbitals in a lower-energy state to orbitals in a higher-energy state. A given molecule can absorb only certain frequencies because only certain states are possible in any molecule and the energy difference between any ground and excited state must be equal to the energy added. At a frequency that is absorbed by a molecule, the intensity of the incident energy is greater than the intensity of the emergent energy, and is a measure of the absorbance. A sample of the fluid being monitored may be compared to a calibration curve (absorbance versus concentration) prepared from standard solutions containing known concentrations of the transition metal (or other suitable tracer specie) to detect and determine the concentration of the tracer. A molecular absorption technique for transition metal tracers is described in U.S. Patent No. 4,992,380, B. E. Moriarity, J. J. Hiekey, W. H. Hoy, I. E. Hoots and D. A. Iohnson, issued February 12, 1991, the disclosure of which is incorporated herein by reference.

A chemical specie may be selected for a given process based on a preference for one or more analytical techniques, or an analytical technique may be selected for a given process based on a preference for one or more chemical tracers. In preferred embodiment, the chemical compound(s) selected as the tracer should be soluble in at least one, and more preferably in both, of the temperature-conditioning fluid and process fluid of the industrial process, at least at the concentration level(s) expected in the responsive fluid. For instance, a tracer maybe fed to a temperature-conditioning fluid and its presence monitored in the process fluid, where its appearance would result only from leakage. The expected concentration in the process fluid upon such leakage occurring would of course be much less than the concentration of such tracer fed to, or maintained, in the temperature-conditioning fluid. If, however, only the decrease of tracer concentration in the fluid to which it is fed is to be monitored, its solubility in the other fluid is generally irrelevant. In preferred embodiment, the chemical compound(s) selected as a tracer should be either stable in the environment of at least one of the fluids, and preferably both of the fluids, for the useful life expected of the tracer, or its loss from the fluid due to degradation, deposition, complexation, or other phenomena should be predictable and compensative, particularly when it is desired not merely to detect the presence of some amount of the tracer, but also to determine the concentration thereof, or change in concentration. In preferred embodiment, the combination of the chemical compound(s) selected as the tracer and
The analytical technique selected for determining the presence and/or concentration of such tracer, should permit such determination(s) without isolation of the tracer, and more preferably should permit such determination(s) on a continuous and/or on-line basis. In preferred embodiment, the analytical technique(s) selected for determining the presence and/or concentration of a tracer, should permit such determination(s) to provide a signal that can activate or regulate the feed of an appropriate treatment chemical(s) to the fluid being contaminated by virtue of the leak detected by the technique.

One embodiment the process of the present invention is comprised of adding a chemical tracer to one, but preferably not both, of the temperature-conditioning fluid and the process fluid, and monitoring the concentration of such tracer in the fluid to which it was added by an analytical technique effective for such tracer, and in preferred embodiment when the same fluid both receives the tracer feed and is subjected to the monitoring, the monitoring is conducted to determine any decrease in the concentration of the tracer in the receiving fluid across at least one potential leakage site, such as a heat-exchanger. In another and preferred embodiment the process of the present invention is comprised of adding a chemical tracer to one, but preferably not both, of the temperature-conditioning fluid and the process fluid, and monitoring the concentration of such tracer in the other fluid by an analytical technique effective for such tracer, and in preferred embodiment when the one fluid receives the tracer feed while the other is subjected to the monitoring, the monitoring is conducted to determine at least any appearance, and more preferably the concentration, of the tracer in the monitored fluid across at least one potential leakage site, such as a heat-exchanger.

In certain embodiments of the invention, the process includes the preliminary steps of sampling the temperature-conditioning and/or the process fluid, preferably at all intended post-tracer-addition sampling sites, to determine baseline conditions. Such preliminary baseline condition(s) determination(s) may also be employed to narrow the selection choice of tracer chemicals or determine and/or modify the selection of post-tracer-addition sampling points. In certain preferred embodiments, the sampling points are at the inlet and outlet of at least one heat-exchanger line, and may be along either or both of the temperature-conditioning and process stream sides of such heat-exchanger. If the suspected leakage or the leakage of greatest concern is from the "A" fluid to the "B" fluid (the A fluid being one of the temperature-conditional fluid or process fluid, and the B fluid being the other), it is feasible to monitor only the A fluid by the "tracer concentration decrease" version of the present invention that is noted above. It would be preferable to instead monitor the B fluid to detect the appearance of the tracer chemical because such monitoring on the side receiving the contamination often would be more sensitive to both the detection and quantification of the leak. Monitoring the B fluid in a given industrial installation may not, however, be the most practical. For instance, the B fluid lines may be less accessible to monitoring than the A fluid lines. The B fluid may provide a less desirable environment for monitoring, or be susceptible to monitoring only by a less desirable method. Thus while the monitoring of the fluid being or in greater jeopardy of being contaminated is a preferred embodiment, circumstances can render that approach less desirable for practical reasons.

By sampling the fluid being monitored at least the outlet, and preferably at both the inlet and outlet, of at least one potential leakage site, and preferably all potential leakage sites, such as at least one heat-exchanger and preferably all heat-exchangers in a water system, one can narrow the potential Icakage locations. For instance, if the B fluid (the contaminated or potentially contaminated fluid) is being monitored at the inlets and outlets of each of a plurality of heat-exchangers arranged in series with a countercurrent flow system, the first outlet appearance of the tracer chemical pinpoints the first heat-exchanger that contains a leak along the direction of the B fluid flowpath, and if the monitoring also includes a determination of the concentration of such tracer and/or the rate of change of such concentration, the magnitude of the leakage at that heat-exchanger site can be calculated using other operating parameters such a flow rates of the respective fluids. An increase in the concentration of the tracer in the B fluid downstream of the first leakage site will pinpoint an addition leakage site, and a determination that the tracer concentration in the B fluid has not increased across a downstream heat-exchanger will remove such heat-exchanger from the suspect category. Further for a countercurrent stream flow system, if the A stream is also monitored the first indication of leakage from the A fluid will occur across its most upstream leaking heat-exchanger, which is the most downstream leaking heat-exchanger for the B stream. Thus if the sensitivity of either monitoring in any way lessens downstream when there are plural leakage sites, in countercurtent flow systems the lessening of monitoring sensitivity in one flow stream is offset by the increasing sensitivity in the other flow stream when both fluids are monitored.

Generally it is desirable to employ the least amount of tracer chemical that is practical for the circumstance, and the amount of the tracer added to the fluid should be at least an amount effective for the determinations desired. Seldom would a tracer be deliberately fed to a fluid in an amount grossly in excess of the minimum effective amount because there generally would be no practical purpose in doing so that would justify the costs involved and any deleterious effects on the quality of either of the fluids caused by the presence of the tracer chemical therein. The amount of tracer chemical to be added to the tracer-receiving fluid that is effective without being grossly excessive will vary with a wide variety of factors, including without limitation the tracer and monitoring method selected, the potential for background interference with the selected monitoring method, the magnitude of the suspected or potential leakage, the monitoring mode (on-line continuous, semi-continuous, slug-and-sample, and the like). Generally the dosage of tracer to one or the other of the fluids will be al least sufficient to provide a concentration of tracer therein of about 0.1 ppm, and more commonly at least about 10 or 100 ppm or higher.

Changes can be made in the composition, operation and arrangement of the method of the present invention described herein without departing from the concept and scope of the invention.

## Claims

1. A method of monitoring a papermaking system containing impurities and containing a dosage of a treating component to determine the level of treating under operating conditions comprising the steps of:
adding to the papermaking system a water soluble fluorescent tracer in an amount proportioned to the amount of treating component in the system, the fluorescent tracer being inert to the papermaking environment;
withdrawing from the system a sample solution containing both the component and tracer and subjecting the withdrawn sample to an analysis which comprises the step of comparing the tracer concentration to a standard to determine the concentration of tracer in the sample; and
changing the volume of water or the dosage of treating component in the papermaking system to maintain an acceptable operating range of concentration for the treating component.

2. The method of claim 1 wherein the step of withdrawing is repeated at different locations in the papermaking system.

3. The method of claim 1 or claim 2 wherein the treating component is a biocide.

4. The method of claim 1, claim 2 or claim 3 wherein the fluorescent tracer consists of 2-napthalensulfonic acid or Acid Yellow 7.

5. A method of controlling the dosage of a treating component in a papermaking system, wherein the treating component has been introduced proportionally with a tracer which is inert to the system, comprising:
providing instrumentation including an analyzer having a flow cell for receiving a sample of circulating water from the papermaking system and for sensing a characteristic of the tracer indicative of its concentration, the analyzer having a transducer for converting the characteristic to a voltage analog thereof; a monitor for receiving said voltage analog and for continuously comparing it to a voltage standard representing a standard tracer in consumption of the treating component, the monitor generating an output signal when the comparison establishes nonstandard performance; and a pump unit for introducing the proportioned treating component and tracer at a predetermined dosage, the output signal controlling the pump unit to alter the dosage of treating component.

6. The method of claim 5 wherein the tracer characteristic is generated by illuminating the sample with electromagnetic radiation.

7. The method of claim 6 wherein the tracer is fluorescent and the sample is passed through a flow cell in the form of a ceramic cylinder, the cylinder being transparent to light and illuminated so emissivity of the tracer may be measured.

8. The method of claim 5, claim 6 or claim 7 wherein the standard concentration of treating component is within predetermined range of 0.5 parts per million to about 100 parts per million, wherein the monitor has adjustable low and high set points which limit and define the range in terms of concentration of proportioned tracer, wherein the monitor generates an output signal to increase the pump unit output when the low setpoint is reached and generates a signal to discontinue the increased pump output when the high setpoint is reached.

9. The method of claim 8 wherein the pump unit comprises a main pump for running constantly to supply a major portion of the treating component tracer dosage, and a trim pump controlled by the signal to supply the remaining dosage.

10. The method of any one of claims 5 to 8 including a sample line for withdrawing an on-stream source of the circulating body of water as a sample, for passing the sample through the flow cell to be analyzed therein, and for returning the analyzed sample to the stream.

11. The method of claim 5 wherein the tracer characteristic is radiant energy generated by illuminating the sample in a photometer.

12. The method of claim 11 wherein the tracer is fluorescent and including the step of passing the sample through a quartz cylinder transparent to and illuminated by light.

13. A process for detecting leakage from a process fluid to a temperature-conditioning fluid in a papermaking system, comprising:
maintaining in the process fluid at least one specie of tracer chemical, which specie of tracer chemical is not a normal component of said temperature-conditioning fluid;
subjecting at least one of said process fluid and said temperature fluid to at least one analysis at least one site; and wherein said analysis at least detects the presence of said specie of tracer chemical and wherein said analysis at least determines the concentration of said specie of tracer chemical when said fluid is subjected to said analysis.

14. The process of Claim 13 wherein said specie of chemical tracer is a synthetically tagged normal component of said process fluid or is foreign to the normal components of said process fluid.

15. The process of Claim 13 wherein said analysis determines a spectral or chemical characteristic of said specie of chemical tracer that is proportional to the concentration of said specie of chemical tracer in the fluid analyzed.

16. The process of Claim 13 wherein at least one of said process fluid and said temperature-onditioning fluid is subjected to analysis across a potential site of leakage.

17. The process of Claim 13 wherein said specie of chemical tracer is at least one fluorescent compound and said analysis at least includes fluorescence and is an on-line analysis.

18. The process of Claim 13 wherein said treatment chemical is a biocide.
